(19) Europäisches Patentamt
European Patent Office
Office européen des brevets

(11) EP 0 571 298 B1

(12) FASCICULE DE BREVET EUROPEEN

(45) Date de publication et mention de la délivrance du brevet: **11.09.1996 Bulletin 1996/37**

(51) Int Cl.6: **C07C 255/07**, C07C 253/30

(21) Numéro de dépôt: **93420184.9**

(22) Date de dépôt: **07.05.1993**

# (54) Procédé de déshydrocyanation de dinitriles aliphatiques

Verfahren zur Dehydrocyanierung aliphatischer Dinitrile

Process for the dehydrocyanation of aliphatic dinitriles

(84) Etats contractants désignés:
**DE ES FR GB NL**

(30) Priorité: **20.05.1992 FR 9206391**

(43) Date de publication de la demande:
**24.11.1993 Bulletin 1993/47**

(73) Titulaire: **RHONE-POULENC FIBER & RESIN INTERMEDIATES**
**92405 Courbevoie (FR)**

(72) Inventeurs:
- **Gubelmann, Michel**
  **F-75016 Paris (FR)**
- **Maliverney, Christian**
  **F-69007 Lyon (FR)**
- **Pernot, Hélène**
  **F-75010 Paris (FR)**

(74) Mandataire: **Vignally, Noel et al**
**RHONE-POULENC CHIMIE,**
**Direction de la Propriété Industrielle,**
**Centre de Recherches des Carrières,**
**B.P. 62**
**69192 Saint-Fons Cédex (FR)**

(56) Documents cités:
**US-A- 2 407 848** **US-A- 3 347 902**

Remarques:
Le dossier contient des informations techniques présentées postérieurement au dépôt de la demande et ne figurant pas dans le présent fascicule.

EP 0 571 298 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

## Description

La présente invention concerne un procédé de déshydrocyanation de dinitriles aliphatiques en phase vapeur.

Elle est plus particulièrement orientée vers la déshydrocyanation de dinitriles aliphatiques saturés ramifiés, qui constituent dans certains cas des sous-produits peu valorisables de la production de dinitriles saturés linéaires. La déshydrocyanation de tels composés permet d'obtenir des nitriles éthyléniques pouvant être réutilisés, notamment dans les procédés de synthèses de dinitriles linéaires.

Le brevet US-A-2 407 848 décrit un procédé de préparation de nitriles éthyléniques par pyrolyse et décomposition thermique de dicyanoalcanes, par passage à une température de 300°C à 500°C environ, sur un catalyseur tel que charbon actif, alumine, bauxite, silice-alumine. Le charbon actif mis en oeuvre dans l'exemple du brevet catalyse la déshydrocyanation du succinonitrile en acrylonitrile. Cependant ce type de catalyseur est inefficace pour déshydrocyaner les dinitriles ramifiés tels que le méthyl-2 glutaronitrile.

Le brevet US-A-3 795 694 décrit l'obtention de 1,4-dicyanobutène et de 2-méthylène-glutaronitrile par déshydrocyanation, généralement en phase liquide, du 1,2,4-tricyanobutane en présence d'un catalyseur basique. Le même brevet décrit également la transformation du 2-méthylène-glutaronitrile en 1-4-dicyanobutène, par l'acide cyanhydrique en présence d'un catalyseur basique.

Un tel procédé ne s'applique pas aux dinitriles aliphatiques ramifiés saturés, qui représentent des quantités parmi les plus importantes de sous-produits disponibles et actuellement généralement détruits.

La présente invention se propose de fournir un moyen de permettre la valorisation de tels sous-produits.

Elle consiste plus précisément en un procédé de déshydrocyanation de dinitriles saturés ramifiés, caractérisé en ce que des vapeurs d'un ou plusieurs desdits dinitriles saturés ramifiés sont mises en contact avec un échangeur de cation acidifié.

Les dinitriles saturés ramifiés qui sont soumis à la déshydrocyanation selon le présent procédé sont plus particulièrement ceux qui répondent à la formule générale (I) :

$$NC-\underset{\substack{| \\ R}}{CH}-(CH_2)_a-CN \qquad (I)$$

dans laquelle :

- R représente un radical alkyle, linéaire ou ramifié, ayant 1 à 4 atomes de carbone,
- a représente un nombre entier de 1 à 6.

Parmi les dinitriles de formule (I), ceux qui sont produits en quantités relativement importantes dans la synthèse industrielle de l'adiponitrile par hydrocyanation du butadiène et dont la valorisation constitue un problème crucial, sont plus particulièrement le 2-méthylglutaronitrile et le 2-éthyl-succinonitrile.

Le procédé de l'invention s'appliquera donc de préférence à ces dinitriles, à leurs mélanges entre eux et à leurs mélanges industriels avec de l'adiponitrile et/ou des pentènes-nitriles.

La température à laquelle est conduit le procédé doit être suffisante pour avoir à l'état de vapeurs les dinitriles saturés à déshydrocyaner.

De préférence elle se situera entre 300°C et 600°C et encore préférentiellement entre 350°C et 500°C.

Une variante préférée de l'inventeur consiste à introduire de l'eau simultanément à l'introduction du ou des dinitriles saturés.

Le rapport molaire eau/dinitrile saturé ramifié varie généralement de 0 à 10 et de préférence de 0 à 5.

Pour faciliter l'introduction du dinitrile saturé, il est commode d'utiliser un gaz vecteur inerte dans les conditions opératoires.

On utilise le plus souvent l'azote, seul ou mélangé avec d'autres gaz inertes.

Le catalyseur mis en oeuvre dans le présent procédé est un échangeur de cation partiellement ou complètement acidifié choisi parmi les solides ayant une capacité échangeuse de cation de 0,001 à 5 milliéquivalents par gramme.

Les échangeurs de cation partiellement ou complètement acidifiés appartiennent essentiellement aux familles suivantes :

- les tamis moléculaires,
- les argiles,
- les argiles à piliers ou argiles pontées,
- les oxydes acides,
- les phosphates lamellaires pontés ou non.

Les tamis moléculaires acides sont plus particulièrement les zéolithes de structure pentasil (comme par exemple la ZSM-5, la ZSM-11, la ZSM-22, la ZSM-23, la ZSM-48, la ferriérite et la mordénite) et les zéolithes de structure faujasite.

Les zéolithes acides de structure pentasil sont préférées dans le cadre de l'invention, notamment celles dans la formule desquelles l'oxyde associé à la silice est celui d'un métal trivalent.

Ce sont plus particulièrement :

- les zéolithes de type ZSM-5 ayant la formule genérale (II) exprimée en termes de rapports d'oxydes :

$$M_{2/n}O, X_2O_3, m\ SiO_2, p\ H_2O \quad (II)$$

dans laquelle :

. M représente un élément choisi parmi l'hydrogène et les métaux mono-, di-, tri- et tétravalents, M étant au moins en partie un atome d'hydrogène,
. X représente un élément trivalent choisi parmi Al, Ga, Fe et B,
. n représente un nombre de 1 à 4,
. m représente un nombre égal ou supérieur à 20,
. p représente un nombre de 0 à 40 ;

- les zéolithes de type ZSM-11 ayant la formule générale (III) exprimée en termes de rapports d'oxydes :

$$M_{2/n}O, Z_2O_3, m\ SiO_2, x\ H_2O \quad (III)$$

dans laquelle :

. M représente un élément choisi parmi l'hydrogène et les métaux mono-, di-, tri- et tétravalents, M étant au moins en partie un atome d'hydrogène,
. Z représente un élément trivalent choisi parmi Al, Ga, Fe et B,
. n représente un nombre de 1 à 4,
. m représente un nombre égal ou supérieur à 20,
. x représente un nombre de 6 à 12.

On préfère généralement les zéolithes dans la formule (II) ou (III) desquelles M est choisi parmi l'hydrogène, les métaux alcalins tels que par exemple Na, K, Li ou Rb, les métaux alcalino-terreux tels que par exemple Be, Mg, Ca, Sr ou Ba, les métaux des terres rares tels que par exemple La ou Ce, les métaux de transition, M étant au moins en partie l'hydrogène.

Pour ces zéolithes, on préférera celles dans la formule (II) ou (III) desquelles les symboles X et Z représentent Al, Ga ou B.

On peut se référer au brevet FR-A-2 622 575 qui est incorporé au présent texte par référence, pour une description plus détaillée des argiles.

On préfère dans le procédé de l'invention utiliser les smectites telles que par exemple les montmorillonites, les beidellites, les nontronites, les hectorites, les stévensites et les saponites.

Les argiles pontées, qui peuvent être utilisées comme catalyseurs dans le présent procédé, sont des argiles entre les feuillets desquelles ont été introduits des ponts ou piliers qui maintiennent un espacement basal. L'espacement basal est la somme de l'épaisseur d'un feuillet de l'argile et de l'espacement interfoliaire.

La préparation de ces argiles pontées a été décrite notamment dans le brevet FR-A-2 543 446 ainsi que dans le brevet FR-A-2 618 143.

Comme argile de départ, on préférera généralement les beidellites.

Le pontage des argiles peut être effectué notamment à l'aide des hydroxydes d'aluminium, de vanadium, de molybdène, de zirconium, de fer, de nobium, de tantale, de chrome, de lanthane, de cérium, de titane, de gallium ou des hydroxydes mixtes de plusieurs de ces métaux.

De préférence, on utilise dans le procédé de l'invention des argiles, particulièrement des beidellites, pontées à l'aide d'hydroxyde d'aluminium.

Les phosphates lamellaires pontés ou non qui peuvent servir de catalyseurs dans le présent procédé sont plus particulièrement des phosphates, des hydrogénophosphates ou des sulfophosphonates de zirconium, de cérium, de thorium, d'uranium, d'hafnium, de plomb, de titane, d'antimoine, de germanium, d'étain ou des mélanges de plusieurs de ces composés.

Les sulfophosphonates métalliques (ou MELS = Molecularly Engineered Layered Structures) sont notamment

ceux qui sont décrits dans le brevet US-A-4 235 990.

Parmi ces composés, on peut citer plus particulièrement les sulfophosphonates de zirconium décrits des REACTIVE POLYMERS, 5 (1987), pages 13 à 21.

Le procédé de l'invention est mis en oeuvre en continu et le débit d'introduction du dinitrile saturé dans l'appareillage est tel que l'on ait de 0,1 à 10 grammes de dinitrile par gramme de catalyseur par heure (c'est la productivité pondérale horaire ou PPH exprimée en $h^{-1}$).

Le temps de contact entre le dinitrile saturé et le catalyseur varie habituellement entre 0,1 seconde et 20 secondes.

Les exemples qui suivent illustrent l'invention.

**<u>EXEMPLES 1 à 5</u>**

Dans un réacteur tubulaire en quartz de 230 mm de longueur et 15 mm de diamètre intérieur, comportant à sa partie inférieure un fritté et équipé d'une gaine contenant 3 thermocouples, de 3 alimentations de fluides (arrivée d'azote et 2 pousse-seringues) et d'un four cylindrique, on introduit successivement sur le fritté :

- des grains de quartz (de diamètre 0,8 à 1,2 mm) sur une hauteur de 1 à 2 cm,
- le catalyseur : 2,31 g (4 $cm^3$) de zéolithe de type ZSM-5 (ayant une capacité échangeuse de cation de 0,63 milliéquivalent $H^+$/g, une formule générale (II) dans laquelle M représente H et X représente Al, un rapport molaire $SiO_2/Al_2O_3$ de 51 et une coupe granulométrique de 0,2 à 1,0 mm),
- une deuxième couche de grains de quartz sur une hauteur de 1 à 2 cm.

Les réactifs sont alimentés à une température de 450°C.

Les débits d'alimentation sont les suivants :

- azote : 1 litre/heure
- 2-méthylglutaronitrile (MGN) : 1 gramme/heure
- $H_2O$ : de 0 à 0,2 gramme /heure

Les paramètres de fonctionnement sont les suivants :

- productivité pondérale horaire (PPH) : 0,43 $h^{-1}$
- temps de contact à 450°C :

  . $MGN/N_2$ 17/83 : 4,9 secondes
  . $H_2O/MGN/N_2$ 17/14/69 : 4,1 secondes.

La mise en régime est de l'ordre de 1 à 2 heures. Les produits sont récupérés à la sortie de l'appareil dans 3 pièges successifs (le premier refroidi à température ambiante, les deux suivants par acétone/glace carbonique).

Le tableau ci-après indique les valeurs des paramètres qui varient selon les essais ainsi que les résultats obtenus.

Dans ce tableau les conventions suivantes ont été utilisés :

tc= temps de contact MGN/catalyseur

TT %= taux de transformation du MGN en %

RR % DHCN= rendement en % de déshydrocyanation (somme de pentène-nitriles et de butène-nitriles obtenus) par rapport au MGN chargé

RR % C= rendement en % de craquage (somme des composés de craquage obtenus) par rapport au MGN chargé

Sel % DHCN= sélectivité en % de la réaction de déshydrocyanation :

$$\frac{RR\ \%\ DHCN}{RR\ \%\ DHCN + RR\ \%\ C}$$

| Exemples | Composition du flux d'alimentation (% molaire) | | | Rapport molaire $H_2O$/MGN | Tc (en sec) | TT % MGN | RR % DHCN | RR % C | Sel % DHCN |
|---|---|---|---|---|---|---|---|---|---|
| | $H_2O$ | MGN | $N_2$ | | | | | | |
| Exemple 1 | 0 | 17 | 83 | 0 | 4,9 | 28 | 3,5 | 6,4 | 35 |
| Exemple 2 | 2 | 17 | 81 | 0,12 | 4,8 | 17 | 6,6 | 7,2 | 48 |
| Exemple 3 | 4 | 17 | 79 | 0,24 | 4,7 | 15 | 7,7 | 5,3 | 59 |
| Exemple 4 | 12 | 15 | 73 | 0,78 | 4,3 | 15 | 9,1 | 3,8 | 71 |
| Exemple 5 | 17 | 14 | 69 | 1,2 | 4,1 | 18 | 13,4 | 4 | 77 |

<u>TABLEAU 1</u>

## Revendications

1. Procédé de déshydrocyanation de dinitriles saturés, ramifiés, caractérisé en ce que des vapeurs d'un ou plusieurs desdits dinitriles saturés ramifiés sont mises en contact avec un échangeur de cation partiellement ou complètement acidifié choisi parmi les solides ayant une capacité échangeuse de cation de 0,001 à 5 milliéquivalents par

gramme.

2. Procédé selon la revendication 1, caractérisé en ce que les dinitriles saturés ramifiés qui sont soumis à la déshydrocyanation sont ceux qui répondent à la formule générale (I) :

$$NC-\underset{\displaystyle R}{\underset{|}{C}H}-(CH_2)_a-CN \qquad (I)$$

dans laquelle :

- R représente un radical alkyle, linéaire ou ramifié, ayant 1 à 4 atomes de carbone,
- a représente un nombre entier de 1 à 6.

3. Procédé selon l'une des revendications 1 ou 2, caractérisé en ce que les dinitriles de formule (I) sont le 2-méthylglutaronitrile, le 2-éthyl-succinonitrile, leurs mélanges entre eux et leurs mélanges industriels avec de l'adiponitrile et/ou des pentènes-nitriles.

4. Procédé selon l'une des revendications 1 à 3, caractérisé en ce que la température à laquelle il est conduit est suffisante pour avoir à l'état de vapeurs les dinitriles saturés à déshydrocyaner, de préférence entre 300°C et 600°C et plus préférentiellement entre 350°C et 500°C.

5. Procédé selon l'une des revendications 1 à 4, caractérisé en ce que l'on introduit de l'eau simultanément à l'introduction du ou des dinitriles saturés.

6. Procédé selon l'une des revendications 1 à 5, caractérisé en ce que le rapport molaire eau/dinitrile saturé ramifié varie de 0 à 10 et de préférence de 0 à 5.

7. Procédé selon l'une des revendications 1 à 6, caractérisé en ce que pour faciliter l'introduction du dinitrile saturé, on utilise un gaz vecteur inerte dans les conditions opératoires, de préférence l'azote, seul ou mélangé avec d'autres gaz inertes.

8. Procédé selon l'une des revendications 1 à 7, caractérisé en ce que les échangeurs de cation acidifiés appartiennent essentiellement aux familles suivantes :

- les tamis moléculaires,
- les argiles,
- les argiles à piliers ou argiles pontées,
- les oxydes acides,
- les phosphates lamellaires pontés ou non.

9. Procédé selon la revendication 8, caractérisé en ce que les tamis moléculaires acides sont des zéolithes de structure pentasil ou faujasite et de préférence les zéolithes acides de structure pentasil, notamment celles dans la formule desquelles l'oxyde associé à la silice est celui d'un métal trivalent.

10. Procédé selon l'une des revendications 8 ou 9, caractérisé en ce que les zéolithes acides de structure pentasil sont :

- les zéolithes de type ZSM-5 ayant la formule générale (II) exprimée en termes de rapports d'oxydes :

$$M_{2/n}O, X_2O_3, m\ SiO_2, p\ H_2O \qquad (II)$$

  dans laquelle :

  . M représente un élément choisi parmi l'hydrogène et les métaux mono-, di-, tri- et tétravalents, M étant au moins en partie un atome d'hydrogène,
  . X représente un élément trivalent choisi parmi Al, Ga, Fe et B,
  . n représente un nombre de 1 à 4,
  . m représente un nombre égal ou supérieur à 20,
  . p représente un nombre de 0 à 40 ;

- les zéolithes de type ZSM-11 ayant la formule générale (III) exprimée en termes de rapports d'oxydes :

$$M_{2/n}O,\ Z_2O_3,\ m\ SiO_2,\ x\ H_2O \qquad (III)$$

dans laquelle :

. M représente un élément choisi parmi l'hydrogène et les métaux mono-, di-, tri- et tétravalents, M étant au moins en partie un atome d'hydrogène,
. Z représente un élément trivalent choisi parmi Al, Ga, Fe et B,
. n représente un nombre de 1 à 4,
. m représente un nombre égal ou supérieur à 20,
. x représente un nombre de 6 à 12 ;

et de préférence celles dans la formule (II) ou (III) desquelles les symboles X et Z représentent Al, Ga ou B.

**11.** Procédé selon l'une des revendications 8 à 10, caractérisé en ce que les zéolithes ont une formule générale (II) ou (III) dans laquelle M est choisi parmi l'hydrogène, les métaux alcalins tels que Na, K, Li ou Rb, les métaux alcalino-terreux tels que Be, Mg, Ca, Sr ou Ba, les métaux des terres rares tels que La ou Ce, les métaux de transition, M étant au moins en partie l'hydrogène.

**Claims**

**1.** Process for the dehydrocyanation of branched saturated dinitriles, characterised in that vapours of one or a number of the said branched saturated dinitriles are brought into contact with a partially or completely acidified cation exchanger chosen from the solids having a cation-exchanging capacity of 0.001 to 5 milliequivalents per gram.

**2.** Process according to claim 1, characterised in that the branched saturated dinitriles which are subjected to the dehydrocyanation are those which correspond to the general formula (I):

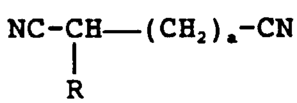

(I)

in which:

- R represents a linear or branched alkyl radical having 1 to 4 carbon atoms,
- a represents an integer from 1 to 6.

**3.** Process according to one of claims 1 or 2, characterised in that dinitriles of formula (I) are 2-methylglutaronitrile or 2-ethylsuccinonitrile, their mixtures with each other and their industrial mixtures with adiponitrile and/or pentenenitriles.

**4.** Process according to one of claims 1 to 3, characterised in that the temperature at which it is carried out is sufficient for the saturated dinitriles to be dehydrocyanated to be in the vapour state, preferably between 300°C and 600°C and more preferentially between 350°C and 500°C.

**5.** Process according to one of claims 1 to 4, characterised in that water is introduced simultaneously with the introduction of the saturated dinitrile(s).

**6.** Process according to one of claims 1 to 5, characterised in that the water/branched saturated dinitrile molar ratio varies from 0 to 10 and preferably from 0 to 5.

**7.** Process according to one of claims 1 to 6, characterised in that, in order to facilitate the introduction of the saturated dinitrile, a carrier gas is used which is inert under the operating conditions, preferably nitrogen, alone or mixed with other inert gases.

**8.** Process according to one of claims 1 to 7, characterised in that the acidified cation exchangers belong essentially

to the following families:

- the molecular sieves,
- the clays,
- the pillared clays or bridged clays,
- the acid oxides,
- the bridged or unbridged lamellar phosphates.

**9.** Process according to claim 8, characterised in that the acidic molecular sieves are zeolites of pentasil or faujasite structure and preferably the acidic zeolites of pentasil structure, especially those of the formula in which the oxide combined with the silica is that of a trivalent metal.

**10.** Process according to one of claims 8 or 9, characterised in that the acid zeolites of pentasil structure are:

- the zeolites of ZSM-5 type having the general formula (II) expressed in terms of ratios of oxides:

$$M_{2/n}O \cdot X_2O_3 \cdot mSiO_2 \cdot pH_2O \qquad (II)$$

  in which:

  - M represents an element chosen from hydrogen and the mono-, di-, tri- and tetravalent metals, M being at least in part a hydrogen atom,
  - X represents a trivalent element chosen from Al, Ga, Fe and B,
  - n represents a number from 1 to 4,
  - m represents a number equal to or greater than 20,
  - p represents a number from 0 to 40;

- the zeolites of ZSM-11 type having the general formula (III) expressed in terms of ratios of oxides:

$$M_{2/n}O \cdot Z_2O_3 \cdot mSiO_2 \cdot XH_2O \qquad (III)$$

  in which:

  - M represents an element chosen from hydrogen and the mono-, di-, tri- and tetravalent metals, M being at least in part a hydrogen atom,
  - Z represents a trivalent element chosen from Al, Ga, Fe and B,
  - n represents a number from 1 to 4,
  - m represents a number equal to or greater than 20,
  - x represents a number from 6 to 12;

and preferably those of formula (II) or (III) in which the symbols X and Z represent Al, Ga or B.

**11.** Process according to one of claims 8 to 10, characterised in that the zeolites have a general formula (II) or (III) in which M is chosen from hydrogen, the alkali metals such as Na, K, Li or Rb, the alkalineearth metals such as Be, Mg, Ca, Sr or Ba, the rare-earth metals such as La or Ce, or the transition metals, M being at least in part hydrogen.

**Patentansprüche**

**1.** Verfahren zur Deshydrocyanierung von verzweigten gesättigten Dinitrilen, dadurch gekennzeichnet, daß Dämpfe eines oder mehrerer dieser verzweigten gesättigten Dinitrile in Kontakt gebracht werden mit einem teilweise oder vollständig sauren Kationenaustauscher, ausgewählt unter den Feststoffen mit einer Kationenaustauscherkapazität von 0,001 bis 5 Milliäquivalent pro Gramm.

**2.** Verfahren gemäß Anspruch 1, dadurch gekennzeichnet, daß die verzweigten gesättigten Dinitrile, welche der Deshydrocyanierung unterworfen werden, solche sind, die der allgemeinen Formel (I):

$$NC - \underset{\displaystyle R}{\underset{|}{CH}} - (CH_2)_a - CN \qquad (I)$$

entsprechen, worin:

- R einen verzweigten oder geraden Alkylrest mit 1 bis 4 Kohlenstoffatomen darstellt,
- a eine ganze Zahl von 1 bis 6 bedeutet.

**3.** Verfahren gemäß einem der Ansprüche 1 oder 2, dadurch gekennzeichnet, daß die Dinitrile der Formel (I) das 2-Methylglutaronitril, das 2-Ethylsuccinotril, deren Mischungen untereinander und deren industrielle Mischungen mit Adiponitril und/oder Penten-nitrile(n) sind.

**4.** Verfahren gemäß einem der Ansprüche 1 bis 3, dadurch gekennzeichnet, daß die Temperatur, bei der es durchgeführt wird, ausreichend ist, um die zu deshydrocyanisierenden gesättigten Dinitrile im Dampfzustand zu haben, vorzugsweise zwischen 300 °C und 600 °C und noch bevorzugter zwischen 350 °C und 500 °C beträgt.

**5.** Verfahren gemäß einem der Ansprüche 1 bis 4, dadurch gekennzeichnet, daß man gleichzeitig mit der Einführung des oder der gesättigten Dinitrils/Dinitrile Wasser einführt.

**6.** Verfahren gemäß einem der Ansprüche 1 bis 5, dadurch gekennzeichnet, daß das Molverhältnis Wasser/verzweigtes gesättigtes Dinitril zwischen 0 bis 10 und vorzugsweise von 0 bis 5 variiert.

**7.** Verfahren gemäß einem der Ansprüche 1 bis 6, dadurch gekennzeichnet, daß man, um die Einführung des gesättigten Dinitrils zu erleichtern, ein Trägergas, das unter den Arbeitsbedingungen inert ist, vorzugsweise Stickstoff, allein oder im Gemisch mit anderen inerten Gasen verwendet.

**8.** Verfahren gemäß einem der Ansprüche 1 bis 7, dadurch gekennzeichnet, daß die sauren Kationenaustauscher im wesentlichen zu den folgenden Familien gehören:

- Molekularsiebe
- die Tone,
- die Säulen- oder Decktone
- die sauren Oxide,
- die lamellaren Phosphate mit Deck oder nicht

**9.** Verfahren gemäß Anspruch 8, dadurch gekennzeichnet, daß die sauren Molekularnetze Zeolithe der Struktur Pentasil oder Faujasit sind und vorzugsweise die sauren Zeolithe der Struktur Pentasil, besonders solche, in deren Formel das mit dem Siliziumdioxid assoziierte Oxid ein solches eines dreiwertigen Metalls ist.

**10.** Verfahren gemäß einem der Ansprüche 8 oder 9, dadurch gekennzeichnet, daß die sauren Zeolithe der Pentasilstruktur sind:

- die Zeolithe vom Typ ZSM-5 mit der allgemeinen Formel (II), ausgedrückt als Verhältnisse der Oxide:

$$M_{2/n}O,\ X_2O_3,\ m\ SiO_2,\ p\ H_2O \qquad (II)$$

worin:

. M ein Element ausgewählt unter Wasserstoff und den ein-, zwei-, drei- und vierwertigen Metallen ist, wobei M mindestens teilweise ein Wasserstoffatom ist,

. X ein dreiwertiges Element, ausgewählt unter Al, Ga, Fe und B darstellt,

. n eine ganze Zahl von 1 bis 4 bedeutet,

- . m eine ganze Zahl gleich oder höher als 20 bedeutet,
- . p eine Zahl von 0 bis 40 darstellt;

- wobei die Zeolithe vom Typ ZSM-11 die allgemeine Formel (III) haben, ausgedrückt als Verhältnis der Oxide:

$$M_{2/n}O,\ Z_2O_3,\ m\ SiO_2,\ x\ H_2O \qquad (III)$$

worin:

- . M ein Element bedeutet, ausgewählt unter Wasserstoff und den ein-, zwei-, drei- und vierwertigen Metallen, wobei M wenigstens zum Teil ein Wasserstoffatom ist,
- . Z ein dreiwertiges Element ausgewählt unter Al, Ga, Fe und B darstellt,
- . n eine ganze Zahl von 1 bis 4 bedeutet,
- . m eine ganze Zahl gleich oder höher als 20 darstellt,
- . x eine ganze Zahl von 6 bis 12 bedeutet;

und vorzugsweise diejenigen, in deren Formel (II) oder (III) die Symbole X und Z Al, Ga oder B bedeuten.

**11.** Verfahren gemäß einem der Ansprüche 8 bis 10, dadurch gekennzeichnet, daß die Zeolithe die allgemeine Formel (II) oder (III) haben, worin M ausgewählt ist unter Wasserstoff, den Alkalimetallen wie Na, K, Li oder Rb, den Erdalkalimetallen wie Be, Mg, Ca, Sr oder Ba, den Seltenerdmetallen wie La oder Ce, den Übergangsmetallen, wobei M mindestens zum Teil Wasserstoff ist.